Europäisches Patentamt

(19) European Patent Office

Office européen des brevets

(11) Publication number: **0 282 554 B1**

# EUROPEAN PATENT SPECIFICATION

(12)

(45) Date of publication of patent specification:
**13.11.91 Bulletin 91/46**

(21) Application number: **87906197.6**

(22) Date of filing: **21.09.87**

(86) International application number:
**PCT/GB87/00658**

(87) International publication number:
**WO 88/01878 24.03.88 Gazette 88/07**

(51) Int. Cl.$^5$: **A61L 15/26**

(54) **ADHESIVE DRESSING.**

The file contains technical information
submitted after the application was filed and
not included in this specification

(30) Priority: **20.09.86 GB 8622695**
**06.12.86 GB 8629231**
**22.01.87 GB 8701434**

(43) Date of publication of application:
**21.09.88 Bulletin 88/38**

(45) Publication of the grant of the patent:
**13.11.91 Bulletin 91/46**

(84) Designated Contracting States:
**AT BE CH DE FR GB IT LI LU NL SE**

(56) References cited:
**EP-A- 80 008**
**EP-A- 147 588**

(73) Proprietor: **SMITH & NEPHEW plc**
**2 Temple Place Victoria Embankment**
**London WC2R 3BP (GB)**

(72) Inventor: **ANSELL, Christopher, Wilson, Guy**
**6 Woodland Road Sawston**
**Cambridge CB2 4DU (GB)**

(74) Representative: **Cole, William Gwyn, Dr.**
**Smith and Nephew Research Limited Gilston**
**Park**
**Harlow Essex CM20 2RQ (GB)**

## Description

The present invention relates to adhesive dressings. More particularly the invention is for dressings utilizing skin-friendly inherently tacky polyurethane gel or foam adhesives such as would be required for wound care applications.

The use of polyurethane - based adhesives for skin application has been proposed, although not for applications where it is necessary for there to be substantially little or no leaching of materials from the adhesive and where the adhesive is required to be skin friendly.

In British Patent Nos. 1468617 and 1351909, there are described adhesive formulations, which may be inter alia based on polyurethanes which contain essentially leachable medicated extracts. Although such adhesives may be tacky, there is no suggestion that such formulations are gel or foam adhesive of inherent tackiness and a low level of leachable materials.

European Patent Specification No. 0147588 discloses self adhesive sheet-like structures comprising a support layer at least partially covered with gel adhesive comprising a cross-linked polyurethane matrix and 38 to 85% by weight of the gel of one or more polyhydroxy compounds, bound by secondary valencies within the matrix as a liquid dispersing agent. Such secondary valency bonding, however, will not prevent leaching of the bound polyhydroxy compounds from the adhesive by water. Gel adhesives of this type therefore contain considerable amount of leachable materials, in some cases more than 20% by weight and would not be suitable for use in wound areas of high exudation.

The present invention seeks to provide an adhesive dressing in which the adhesive is skin friendly ie. causes no tendency to strip skin layers when removed, leaves no deposits, and yet can be employed for direct wound contact and contains substantially no leachable materials.

An adhesive dressing suitable for application to skin comprising a backing layer and a layer of a polyurethane gel or foam adhesive which adhesive is inherently tacky and is obtained by reacting a prepolymer formed as the reaction product of a polyisocyanate having a functionality of from 2.1 to 5 and polyoxyalkylene diol monoalkyl ether having a molecular weight of from 3000 to 12000 and wherein the mole ratio NCO/OH is from 2 to 4 with a hydroxyl-containing compound comprising water or a polyol wherein the final polymer contains not more than 10% by weight of water leachable materials.

Tack is a property of an adhesive whereby light contact with the surface of another body brings about a condition requiring force to restore the original separated state, that is a property which will inhibit but not wholly prevent the removal or a smooth contacting surface such as a metal probe or a human finger. By inherent tack means the gel or foam possesses this property without requiring the addition of any further adhesion promoting component such as a tackifier. Tack and how it may be measured are discussed in Chapter 3 of Handbook of Pressure-Sensitive Adhesive Technology Editor D. Satas, Van Nostrand Reinhold Co. Inc. New York, 1982.

Suitably the inherently tacky gel is a pressure sensitive adhesive, that is the adherency of the gel is dependent upon the force used to apply it to a surface. Other types of adhesive which comprise inherently tacky gels of highly hydrophilic polyurethane and inherently tacky foams of highly hydrophilic polyurethane are however, also within the scope of this invention.

The dressings of the invention are further characterised in that the contents of materials leachable into water will be no greater than 10% by weight of the adhesive. More suitably the content of materials leachable into water will be less than 5%, preferably less than 2.5% by weight of the adhesive. The adhesives used in the dressing of the invention do not require the presence of additives such as humectants or plasticizers which may leach out into a wound surface and thus be considered generally unsatisfactory. Thus "leachable materials" as used herein are materials which are leachable by water from the adhesive of the dressing of the invention and which would be considered unsatisfactory for use on a wound surface.

The adhesive employed in the dressings of the invention may be hydrophilic polyurethanes. One class of highly hydrophilic polyurethanes are those prepared by reacting water or a polyol with an isocyanate prepolymer which is the reaction product of a polyfunctional isocyanate and a polyoxyalkylene diol monoalkyl ether.

By highly hydrophilic is meant that the polyurethane when hydrated may contain from 35 to 95% by weight of water, aptly 50 to 92%, preferably 70 to 90% and more preferably 75 to 85% by weight of water. Water absorption can be obtained by taking a known weight of the polyurethane and immersing in water for 24 hours. The hydrated polymer is removed from the water, excess water is removed by lightly blotting with absorbent paper and then the hydrated polyurethane is weighed. The water absorption of the polyurethane (% by weight) can then be calculated as

2

(weight of hydrated polyurethane - weight of dry polyurethane)x10

weight of hydrated polyurethane

The inherently tacky adhesive gels or foams may be formed by mixing a prepolymer formed as the reaction product of polyisocyanate and polyoxyalkylene diol mono alkyl ether with a molar excess of water or with a stoichiometric amount of polyol or polyol mixture. The polyol is preferably a diol or a mixture of diols. The excess of water used in absorbed by the tacky gel or foam produced.

In addition to the water or polyol or a mixture thereof, at least a portion of hydroxyl compound may be a hydroxyl compound which is capable of reacting with isocyanate and which contains an unsaturated functionality which is reactive when exposed to irradiation.

Thus, another class of adhesives suitable for use in the present invention are polyurethane adhesives which also contain acrylate or methacrylate residues in addition to the polyurethane residues. Such adhesives may be prepared by reacting an isocyanate prepolymer, which may itself be the reaction product of a polyfunctional isocyanate and a polyoxy alkylene diol monoalkyl ether, with a hydroxy containing compound, e.g. water or a polyol and a hydroxyl - containing ester of acrylic or methacrylic acid and/or mixtures of such hydroxyl - containing compounds.

Suitable polyoxyalkylene diol mono alkyl ethers for use in forming the prepolymers employed to prepare the adhesive employed in the present invention include those in which the alkyl group contains from 1 to 18 carbon atoms and more suitably 2 to 12 carbon atoms and preferably 2 to 6 carbon atoms for example 4 carbon atoms that is the mono alkyl ether is a monobutyl ether.

Suitably the polyoxyalkylene residue in the mono alkyl ether is a hydrophilic residue that contains polyoxyethylene or polyoxypropylene residues or mixtures thereof. Preferred polyoxyalkylene residues are those which contain a mixture of polyoxyethylene and polyoxypropylene residues in the ratio of from 20:80 to 80:20 for example 50:50. The residues may be randomly arranged with respect to each other.

A preferred polyoxyalkylene diol mono alkyl ether is therefore polyoxyethylene - polyoxypropylene mono butyl ether in which the ratio of polyoxyethylene to polyoxypropylene residues is about 50:50 such as the Emkarox polyols sold by I.C.I. plc.

Aptly the molecular weight of the monoalkyl ether is from 3000 to 12000, and more suitably is 4000 to 10000 and preferably is 5000 to 9000. It has been found that polyoxyalkylene diol monoalkyl ethers within this molecular weight range provide polymers which are tacky and suitable for use on the skin while use of lower molecular weight materials tend to provide polymers which are not suitable for use as adhesives because they are insufficiently tacky.

The polyoxyalkylene diol mono alkyl ethers for use in forming the prepolymer will normally contain water. It is preferred however that the polyoxyalkylene diol monoalkyl ether contains less than 1% by weight of water so as to limit the proportion of urea groups formed in the reaction with the polyisocyanate.

The polyisocyanate used for forming the prepolymer will have a functionality of greater than $2^-$ and may suitably have a functionality of from 2.1 to 5 and more suitably from 2.2 to 3.5 and preferably from 2.5 to 3.0 for example 2.5, 2.7, 2.85 or 2.9. Suitable polyisocyanate include (cyclo) aliphatic and aromatic polyisocyanates. Preferred polyisocyanates are aromatic polyisocyanates for example those based on a polymeric methylene diphenyl diisocyanate, for example the Suprasecs (trade mark), which are available from I.C.I.. It is preferred that the functionality of the isocyanate is not more than 3 as this leads to a higher cross-link density and is manifest ultimately as a harder adhesive which may not be advantageous for an adhesive which is to be applied to the skin.

The prepolymers may be simply prepared by heating the two components together in the required proportions at an elevated temperature for sufficient time for the reaction to be completed, for example 90°C for 2 hours, in the presence of 0.2% w/w catalyst of dibutyl tin dilaurate (T12). The mole ratio NCO/OH is suitably from 2.0 to 4.0 in this reaction.

The adhesive based solely on polyurethanes may be prepared by reacting the prepolymer described above with water. The reaction mixture may contain from 10% w/w water and 90% w/w prepolymer to 90% w/w water and 10% w/w prepolymer. It is preferred, however, when forming a tacky gel that the concentration of prepolymer is less than 25% w/w. If the concentration of prepolymer is greater than 25% w/w then a tacky foam is produced, which also forms part of this invention. Normally the reactants are mixed together at room temperature and allowed to cure for at least 30 minutes. The excess water is taken up into the tacky gel or foam to produce a swollen gel or foam. It has been found that these gels and foams may take up a further amount of water often upto a weight equal to their original weight.

The polyurethane adhesives employed in the present invention may also be prepared by reacting the prepolymer with a stoichiometric amount of polyol. The reactants are mixed together and cured at elevated tem-

3

perature for 15 minutes to 2 hours. The resultant polyurethane polymer is in the form of a dry adhesive mass which is inherently tacky and may absorb upto 90% by weight of water. Suitably the polyol is a diol. The choice of the polyol and isocyanate-content of the prepolymer govern the degree of tackiness of the resulting adhesive. Thus the lower isocyanate-content of the prepolymer, the lower is the cross-link density of the resultant polymer and the more tacky is the adhesive. Thus, also the higher the molecular weight of the polyol the softer is the adhesive mass.

Suitable polyols are diols which include alkylene glycols, di-alkylene glycols and polyoxyalkylene diol block copolymers. Suitable diols include diethylene glycol, polyoxyethylene diol having a molecular weight of about 200 and a polyoxyethylene-polyoxypropylene diol block copolymer having a molecular weight of about 2600, and polyoxypropylene diol of molecular wt about 2000.

The polyurethane-acrylate adhesives employed in the present invention may be prepared by reacting the prepolymer described above with hydroxy-containing compounds a proportion of which also contains an unsaturated functionality which is reactive when exposed to irradiation for example ultra violet or electron beam irradiation.

Suitable unsaturated compounds include esters of acrylic or methacrylic acid in which there is at least one hydroxyl functional group which is capable of reacting with isocyanate present. Preferred esters include hydroxy ethyl methacrylate and methacrylate mono-esters of of polyoxylalkylene diols in which the number of repeating ether units is from 1 to 25 and preferably 2 to 10 for example 6.

The remainder of the hydroxyl-containing compounds comprise polyols such as diols or triols or mono-ols such as primary alcohols or polyoxyalkylene monoalkyl ethers as herein before described. Preferably the hydroxyl-containing compounds are polyoxyalkylene monoalkyl ethers or other mono-ols which may confer additional tacky properties to the adhesive. Such mono-ols include hydrogenated mono hydroxyl tackifying resins such as hydrogenated abietyl alcohol.

The proportion of hydroxyl-containing compound which also contains an unsaturated functionality is such as to react with from 15 to 25% of the free isocyanate groups in the prepolymer. The remainder of the free isocyanate groups react with the other hydroxyl-containing compounds.

The resulting polymer has therefore pendant unsaturated groups which are capable of interacting with each other to cross-link the polymer under the influence of a polymerisation initiator and irradiation. One suitable form of radiation is ultraviolet irradiation. The polymer may be mixed with a photoinitiator and the mixture irradiated by means of ultraviolet radiation. This causes the polymer to become cross-linked. The polyurethane polymer so formed is a cross-linked polymer which is capable of absorbing from 5 to 95% by weight of water depending upon the reactants employed.

Alternatively the adhesive may be formed as a 'wet' adhesive by dispersing the uncross-linked polymer and photoinitiator in an appropriate volume of water and then irradiating to form the adhesive. Suitably the wet adhesive may contain from 40 to 65% by weight of water.

The backing or supporting layers employed in the present invention should be capable of conforming to the body contours when applied to the skin and should be flexible enough to move with the body without becoming detached. Suitable flexible backing materials include knitted, woven or non-woven fabrics, nets, microporous films such as plasticised polyvinyl chloride films, polymer blend films containing voids, polymeric films, including thermoplastic polyurethane and hydrophilic polyurethane, elastomeric polyesters, styrene-butadiene block copolymers such as Kraton (Trade mark) thermoplastic rubbers.

Favoured materials which may be used as the backing layer in the dressing embodiment of the present invention include films of hydrophilic polymers. Apt hydrophilic polymers include hydrophilic polyurethanes, polyvinyl pyrrolidone, polyvinyl alcohol and cellulosic derivatives.

A favoured hydrophilic polymer is a hydrophilic polyurethane. Suitable hydrophilic polyurethanes include those having the composition and prepared by the process described in British Patent no. 2093190B. The most suitable hydrophilic polyurethanes are those which contain from 5 to 50% by weight of water when hydrated move suitably to 10 to 40% by weight of water and which have a thickness when present in a dressing of from 25 to 120μm, more suitably 30 to 60μm. A preferred film of hydrophilic polyurethane has a water content when hydrated of 20 to 30% for example 25% and a thickness of 50μm for example 30μm.

A favoured flexible backing material is a microporous plasticised polyvinyl chloride film formed by the process disclosed in British Patent No. 884232. The preferred microporous plasticised polyvinyl chloride 30 to films have a thickness of from 100 to 300 μm, for example 150μm, 200μm and 250μm.

A further favoured backing layer is a thermoplastic polyurethane including the linear polyester polyurethanes or polyether polyurethanes known as Estanes (Trade mark). Such polyurethanes are used as films from 15 to 75 μm in thickness, more favourably 20 to 35μm in thickness for example about 25μm or 30μm.

The adhesive layer may have a thickness of from 0.05 to 5mm depending upon the type of skin lesion, wound or other similar use the dressing is to be put, that is thinner layers may be used on non-exuding wounds or on dressings for use at intravenous sites while thicker layers may be used on exuding wounds where the absorptive properties of the adhesise may be advantageously used. More suitably the adhesive layer may have a thickness of 1.5 to 4mm and preferably 2 to 3mm.

The dressings of the present invention may be prepared by casting the backing layer from a solution of the appropriate polymer at a suitable concentration onto a silicone release paper and removing the solvent to give a film of the required thickness. A film of the adhesive at the required thickness is prepared and the two films brought together and adhered to each other to form a laminate, preferably without the use of any further adhesives. A release coated protector may be placed over the adhesive layer and the laminate cut into dressings of the appropriate size for example 5cm x 5cm, 7.5cm x 7.5cm, 8cm x 8cm, 10cm x 10cm, 5cm x 10cm and 10cm x 20cm. The dressings may be packaged in a bacteria-proof and water-proof pouch and be sterilized by conventional methods such as irradiation and ethylene oxide.

An adhesive dressing of the present invention can be used for treating a wound on an animal body by applying the dressing to the wound.

In use the sterile dressing is removed from the pouch, the protector removed from the adhesive surface of the dressing and the dressing is applied to the wound.

The dressing may be used as a dressing for wounds caused by physical or surgical trauma, burns, ulcers and the like, as a surgical drape, as a dressing for intravenous access sites and any dressing for which long term attachment to the skin may be required.

Many medicinal agents may be incorporated into the adhesives of the present invention. By medicinal agent it is meant pharmacologically active agents and agents including topical anaesthetics such as xylocaine, bacteriostatic agents such as silver nitrate; antibacterial agents of which preferred agents are silver sulphadiazine and chlorhexidine salts; antibiotics; topical steroids, enzymes, tissue stimulants, coagulants and anticoagulants and antifungal agents. Other agents such as emollients may be added after the reaction step.

Advantageously water soluble medicaments such as chlorhexidine and its salts may be dissolved in the water which is used to react with the prepolymer. It is found that chlorhexidine is unaffected during the process and the resulting adhesive provides effective release of chlorhexidine when placed onto the skin.

Example 1

A polyoxyethylene - polyoxypropylene diol monobutyl ether, which has a ratio of polyoxyethylene to polyoxypropylene residues of 1:1 and molecular weight of 4095 (300g, 0.073 moles based on OH value) and a polymeric methylene diphenyl diisocyanate (37.21g, 0.266 moles, - NCO functionality of 2.7) were mixed together at an NCO/OH ratio of 2.5 in a 700cm$^3$ flange flask fitted with an overhead stirrer. The flask was heated in a water bath set to a temperature of 90°C. A catalyst comprising dibutyl tin dilaurate (0.2% w/w) was added. The mixture was stirred at 90°C for two hours. The prepolymer so formed was allowed to cool. The prepolymer was a golden yellow viscous liquid which may be stored in a capped bottle until ready use. The prepolymer was found to have isocyanate content of 1.98%.

A portion of the prepolymer and the calculated quantity of diethylene glycol which would react with all the available isocyanate were mixed at room temperature until homogeneous and then spread onto a silicone release paper at a weight per unit area of 280g/m$^2$ and cured at 90°C to give an adhesive mass.

The adhesive mass contained 85% by weight of water when fully hydrated.

A precast film of a thermoplastic polyether polyurethane may be transfer coated onto the adhesive mass and the laminate strip so formed cut into pieces which are suitable for adhesive dressings and packed in a bacteria proof and waterproof package and sterilised by irradiation.

In use the sterile dressing is removed from the package, the silicone coated release paper removed and the adhesive layer placed against and around the wound area.

A film of adhesive gel was coated onto various substrates commonly used in wound dressings at a weight per unit area of either 285g/m$^2$ or 70g/m$^2$. The upright and inverted moisture vapour transmission rates were measured and the results shown in the following table.

Demonstration 1

Upright MVP Demonstration

Discs of material under test are clamped over Payne Permeability Cups (flanged metal cups using sealing rings and screw clamps. The exposed surface area of the test sample may be conveniently 10cm$^2$. Each cup contains approximately 10ml of distilled water.

After weighing the cups are placed in a fan assisted oven which is maintained at 37 ± 1°C. The relative humidity within the oven is maintain at approximately 10% by placing 1Kg of anhydrous 3-8 mesh calcium chloride on the floor of the oven.

The cups are removed after 24 hours, allowed to cool for 20 minutes and re-weighed. The MVP of the test material is calculated from the weight loss experienced in units of grams of weight per square metre per 24 hours.

Inverted MVP Determination

The method described above is employed except that the Payne Cups are inverted in the oven so that the water within the cups is in contact with the test material and in this case with the gel adhesive.

| Substrate | Weight per unit area of adhesive | Upright (U)/ Inverted cup (I) | MVTR (g/m$^2$/24h) |
|---|---|---|---|
| Polyurethane containing 25% by weight water when hydrated, 30gsm | 285 285 | U I | 1472 7356 |
| Polyurethane (as above) | 70 70 | U I | 1455 >14000 |
| Plasticised Polyvinyl Chloride (150um thick) | 70 70 | U I | 1444 5384 |
| Knitted Fabric | 70 70 | U I | 5600 >14000 |
| Polymer net prepared according to UK Patent No. 1531715 | 70 70 | U I | 1644 >14000 |

7

## Claims

1. An adhesive dressing suitable for application to skin comprising a backing layer and a layer of a polyurethane gel or foam adhesive which adhesive is inherently tacky and is obtained by reacting a prepolymer formed as the reaction product of a polyisocyanate having a functionality of from 2.1 to 5 and polyoxyalkylene diol monoalkyl ether having a molecular weight of from 3000 to 12000 and wherein the mole ratio NCO/OH is from 2 to 4 with a hydroxyl-containing compound comprising water or a polyol wherein the final polymer contains not more than 10% by weight of water leachable materials.

2. A dressing as claimed in claim 1 wherein the polyurethane is a hydrophilic polyurethane.

3. A dressing as claimed in claim 2 wherein, when hydrated, the polyurethane contains 35 to 95% by weight water.

4. A dressing as claimed in claim 3 wherein, when hydrated the polyurethane contains from 75 to 85% by weight water.

5. A dressing as claimed in any one of the preceding claims wherein the adhesive contains residues derived from both polyurethane and an acrylate or methacrylate.

6. A dressing as claimed in any one of the preceding claims wherein at least a proportion of the hydroxyl compound is a hydroxyl compound which is capable of reacting with isocyanate and which contains an unsaturated functionality which is reactive when exposed to irradiation.

7. A dressing as claimed in claim 6 wherein the hydroxyl compound is an ester of acrylic or methacrylic acid.

8. A dressing as claimed in any one of the preceding claims wherein the concentration of water is from 10 to 90% by weight of the prepolymer-water reaction mixture.

9. A dressing as claimed in claim 8 wherein the prepolymer is less than 25% by weight of the prepolymer-water reaction mixture.

10. A dressing as claimed in any one of the preceding claims wherein the polyol is a diol.

11. A dressing as claimed in claim 10 wherein the diol is present in a stoichiometric amount with respect to the isocyanate prepolymer.

12. A dressing as claimed in any one of the preceding claims wherein the alkyl moiety of the polyoxyalkylene monalkyl ether contains from 2 to 6 carbon atoms.

13. A dressing as claimed in any one of the preceding claims wherein the polyoxyalkylene residue of the ether is a hydrophilic residue containing polyoxyethylene or polyoxypropylene residues or mixtures thereof.

14. A dressing as claimed in any one of the preceding claims wherein the ether is a polyoxyethylene - polyoxypropylene mono-butyl ether in which the ratio of polyoxyethylene to polyoxypropylene residues is about 50:50.

15. A dressing as claimed in any one of the preceding claims wherein the support layer is a flexible knitted, woven or non-woven fabric, a net, a microporous polymer film, or a moisture vapour permeable polymeric film.

16. A dressing as claimed in any one of the preceding claims wherein the adhesive contains a pharmacologically active medicinal agent.

17. A dressing as claimed in any one of the preceding claims wherein the content of materials leachable into water is less than 2.5% by weight of the adhesive.

18. A dressing as claimed in any one of the preceding claims, within a sterile package.

## Patentansprüche

1. Zur Anbringung auf die Haut geeignetes Heltpflaster, umfassend eine Trägerschicht und eine Schicht aus einem Polyurethan-Gel- oder -Schaum-Klebestoff, welcher schon an sich klebrig ist und welcher durch Umsetzen eines Präpolymers, welches als das Reaktionsprodukt eines Polyisocyanats mit einer Funktionalität von 2,1 bis 5 und einem Polyoxyalkylendiol-monoalkyl-ether mit einem Molekulargewicht von 3000 bis 12000 gebildet wurde und wobei das Molverhältnis NCO/OH 2 bis 4 ist, mit einer hydroxylhaltigen Verbindung, welche Wasser oder ein Polyol umfaßt, erhalten wird, wobei das Endpolymer nicht mehr als 10 Gew.-% durch Wasser auslaugbare Materialien enthält.

2. Pflaster wie in Anspruch 1 beansprucht, worin das Polyurethan ein hydrophiles Polyurethan ist.

3. Pflaster wie in Anspruch 2 beansprucht, worin das Polyurethan, wenn es gewässert wird, 35 bis 95 Gew.-% Wasser enthält.

4. Pflaster wie in Anspruch 3 beansprucht, worin das Polyurethan, wenn es gewässert wird, 75 bis 85 Gew.-

% Wasser enthält.

5. Pflaster wie in einem der vorangehenden Ansprüche beansprucht, worin der Klebestoff sowohl von einem Polyurethan als auch einem Acrylat oder Methacrylat abgeleitete Reste enthält.

6.Pflaster, worin wenigstens ein Teil der Hydroxyl-Verbindung eine Hydroxyl-Verbindung ist, welche befähigt ist, mit einem Isocyanat zu reagieren, und welche eine ungesättigte Funktionalität enthält, welche reaktiv ist, wenn sie einer Bestrahlung ausgesetzt wird.

7. Pflaster wie in Anspruch 6 beansprucht, worin die Hydroxyl-Verbindung ein Ester der Acryl- oder Methacrylsäure ist.

8. Pflaster wie in einem der vorangehenden Ansprüche beansprucht, worin die Konzentration an Wasser 10 bis 90 Gew.-% des Präpolymer-Wasser-Reaktionsgemisches beträgt.

9. Pflaster wie in Anspruch 8 beansprucht, worin das Präpolymer weniger als 25 Gew.-% des Präpolymer-Wasser-Reaktionsgemisches beträgt.

10. Pflaster wie in einem der vorangehenden Ansprüche beansprucht, worin das Polyol ein Diol ist.

11. Pflaster wie in Anspruch 10 beansprucht, worin das Diol in stöchiometrischer Menge bezogen auf das Isocyanat-Präpolymer vorhanden ist.

12. Pflaster wie in einem der vorangehenden Ansprüche beansprucht, worin die Alkylhälfte des Polyoxyalkylen-monoalkyl-ethers 2 bis 6 Kohlenstoff-Atome enthält.

13. Pflaster wie in einem der vorangehenden Ansprüche beansprucht, worin der Polyoxyalkylen-Rest des Ethers ein hydrophiler Rest ist, welcher Polyoxyethylen- oder Polyoxypropylen-Reste oder deren Gemische enthält.

14. Pflaster wie in einem der vorangehenden Ansprüche beansprucht, worin der Ether ein Polyoxyethylen-polyoxypropylen-monobutylether ist, bei welchem das Verhältnis von Polyoxyethylen- zu Polyoxypropylen-Resten etwa 50:50 beträgt.

15. Pflaster wie in einem der vorangehenden Ansprüche beansprucht, worin die Trägerschicht ein biegsamer gewirkter oder nicht-gewirkter Webstoff, ein Maschenwerk, ein mikroporöser Polymerfilm oder ein feuchtigkeitsdampfdurchlässiger Polymerfilm ist.

16. Pflaster wie in einem der vorangehenden Ansprüche beansprucht, worin der Klebestoff ein pharmakologisch wirksames, medizinisches Mittel enthält.

17. Pflaster wie in einem der vorangehenden Ansprüche beansprucht, worin der Gehalt an in Wasser auslaugbare Materialien weniger als 2,5 Gew.-% des Klebestoffs beträgt.

18. Pflaster wie in einem der vorangehenden Ansprüche beansprucht in einer sterilen Verpackung.


## Revendications

1. Pansement adhésif pouvant être appliqué sur la peau comprenant une couche de support et une couche d'un adhésif sous forme de gel ou de mousse de polyuréthane, lequel adhésif manifeste une adhérence immédiate propre et est obtenu par réaction d'un prépolymère formé en tant que produit réactionnel d'un polyisocyanate ayant une fonctionnalité de 2,1 à 5 et d'un éther monoalkylique de poly-oxyalkylènediol ayant un poids moléculaire de 3000 à 12000 et dans lequel le rapport molaire NCO/OH est de 2 à 4 avec un composé contenant une fonction hydroxy comprenant de l'eau ou un polyol, dans lequel le polymère final ne contient pas plus de 10 % en poids de matières pouvant être lessivées dans l'eau.

2. Pansement suivant la revendication 1, caracterisé en ce que le polyuréthane est un polyuréthane hydrophile.

3. Pansement suivant la revendication 2, caractérisé en ce que lorsqu'il est hydraté, le polyuréthane contient 35 à 95 % en poids d'eau.

4. Pansement suivant la revendication 3, caractérisé en ce que lorsqu'il est hydraté, le polyuréthane contient de 75 à 85 % en poids d'eau.

5. Pansement suivant l'une quelconque des revendications précédentes, caractérisé en ce que l'adhésif contient des résidus dérivés à la fois de polyuréthane et d'un acrylate ou d'un méthacrylate.

6. Pansement suivant la revendication 1, caractérisé en ce qu'au moins une proportion du composé hydroxy est un composé hydroxy qui est capable de réagir avec un isocyanate et qui contient une fonctionnalité insaturée qui peut réagir lorsqu'elle est exposée à une irradiation.

7. Pansement suivant la revendication 6, caractérisé en ce que le composé hydroxy est un ester d'acide acrylique ou méthacrylique.

8. Pansement suivant les revendications précédentes, caractérisé en ce que la concentration de l'eau est de 10 à 90 % en poids du mélange réactionnel de prépolymère-eau.

9. Pansement suivant la revendication 8, caractérisé en ce que le prépolymère représente moins de 25 % en poids du mélange réactionnel de prépolymère-eau.

10. Pansement suivant les revendications précédentes, caractérisé en ce que le polyol est un diol.

11. Pansement suivant la revendication 10, caractérisé en ce que le diol est présent en une quantité stochiométrique par rapport au prépolymère d'isocyanate.

12. Pansement suivant les revendications précédentes, caractérisé en ce que la partie alkyle de l'éther mono-alkylique du polyoxyalkylène contient de 2 à 6 atomes de carbone.

13. Pansement suivant les revendications précédentes, caractérisé en ce que le résidu polyoxyalkylène de l'éther est un résidu hydrophile contenant des résidus polyoxyéthylène ou polyoxopropylène ou leurs mélanges.

14. Pansement suivant les revendications précédentes, caractérisé en ce que l'éther est un éther monobutylique de polyoxyéthylène-polyoxypropylène dans lequel le rapport des résidus polyoxyéthylène à polyoxypropylène est d'environ 50/50.

15. Pansement suivant l'une quelconque des revendications précédentes, caractérisé en ce la couche de support est un tissu tissé ou non-tissé tricoté flexible, un filet, un film de polymère microporeux ou un film polymère perméable à la vapeur d'eau.

16. Pansement suivant l'une quelconque des revendications précédentes, caractérisé en ce que l'adhésif contient un agent médicinal actif du point de vue pharmacologique.

17. Pansement suivant l'une quelconque des revendications précédentes, caractérisé en ce que la teneur en matières pouvant être lessivées dans l'eau est inférieure à 2,5 % en poids de l'adhésif.

18. Pansement suivant l'une quelconque des revendications précédentes, caractérisé en ce qu'il se trouve dans un conditionnement stérile.